# EUROPÄISCHE PATENTSCHRIFT

(11) **EP 0 894 830 B1**
(45) Veröffentlichungstag und Bekanntmachung des Hinweises auf die Patenterteilung: **24.08.2011**
(21) Anmeldenummer: 98810637.3
(22) Anmeldetag: 07.07.1998
(51) Int. Cl.: C09B 29/085, C07C 233/43, C09B 67/38, D06P 3/54

(54) **Dispersionsfarbstoffe**
Dispersion dyestuffs
Colorants de dispersion

(30) Priorität: 15.07.1997 CH 172597
(43) Veröffentlichungstag der Anmeldung: 03.02.1999
(73) Patentinhaber: Huntsman Advanced Materials (Switzerland) GmbH, 4057 Basel (CH)
(72) Erfinder: Herzig, Paul, 4057 Basel (CH); Clément, Antoine, 4058 Basel (CH); Arquint, Alfons, 4058 Basel (CH)

(56) Entgegenhaltungen:
- EP-A- 0 555 179
- FR-A- 1 377 496
- JP-A- 58 152 056
- CHEMICAL ABSTRACTS, vol. 94, no. 10, 9. März 1981 Columbus, Ohio, US; abstract no. 67273a, "Azo dyes" Seite 87; XP002019643 -& JP 55 116754 A (MITSUI TOATSU CHEMICALS, INC.) 8. September 1980

## Beschreibung

Gegenstand der vorliegenden Erfindung sind Dispersionsfarbstoffe, Verfahren zu deren Herstellung sowie ihre Verwendung zum Färben oder Bedrucken von halbsynthetischen oder synthetischen hydrophoben Fasermaterialien.

Dispersionsfarbstoffe, d.h. Farbstoffe, welche keine wasserlöslichmachenden Gruppen enthalten, sind seit langem bekannt und werden zum Färben von hydrophoben Textilmaterial verwendet.

Monoazofarbstoffe erhältlich durch Kupplung von diazotierten 4-Nitro-2-halogenanilinen und N,N-Bis(alkoxycarbonylalkyl)anilinen, die zum Färben von Polyesterfasern geeignet sind, werden in der JP-A 55-116754 und der FR-A 1 377 496 beschrieben.

Vielfach sind die erhaltenen Färbungen jedoch nicht ausreichend thermomigrierecht und haben auch zum Teil unbefriedigende Echtheiten, insbesondere Wasch- und Schweissechtheit. Dieses Problem tritt vor allem bei blauen und marineblauen Nuancen auf.

Gegenstand der vorliegenden Erfindung sind nun Dispersionsfarbstoffe, welche sehr thermoigrierechte und wasch- und schweissechte Färbungen ergeben, und zu dem sowohl im Auszieh- und Thermosolverfahren als auch im Textildruck ein gutes Aufbauvermögen besitzen. Die Farbstoffe sind auch für den Ätzdruck geeignet.

Die erfindungsgemässen Farbstoffe entsprechen der Formel 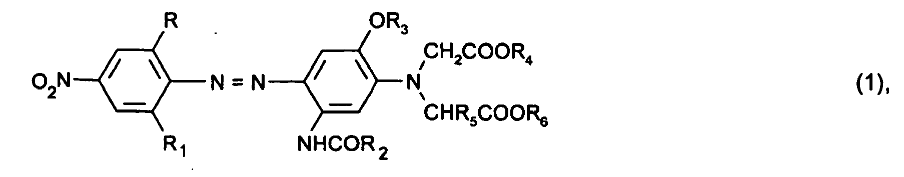 worin R Nitro oder Cyano, R₁ Halogen, R₂ unsubstituiertes, oder durch C₁-C₃-Alkoxy, Halogen, Cyano oder Phenyl substituiertes C₁-C₄-Alkyl, R₃ C₁-C₄-Alkyl, R₄ Methyl oder Ethyl, R₅ Methyl und R₆ Methyl oder Ethyl sind.

R₁ als Halogen ist Brom, Chlor oder Jod.

R₂ und R₃ als C₁-C₄-Alkyl sind unabhängig voneinander z.B. Methyl, Ethyl, n-Propyl, Isopropyl, n-Butyl, sec.-Butyl, tert.-Butyl und Isobutyl.

Als R₁ ist Chlor oder Brom und insbesondere Chlor bevorzugt.

Als R₂ ist Ethyl oder Methyl und insbesondere Methyl bevorzugt.

Als R₃ ist Ethyl oder Methyl und insbesondere Methyl bevorzugt.

Die Farbstoffe der Formel (1) können nach an sich bekannten Verfahren hergestellt werden. Man erhält sie beispielsweise, indem man eine Verbindung der Formel 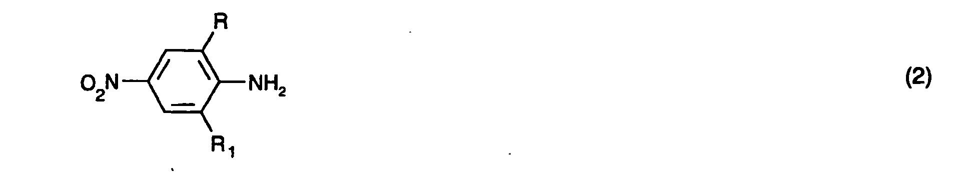 diazotiert und auf eine Kupplungskomponente der Formel 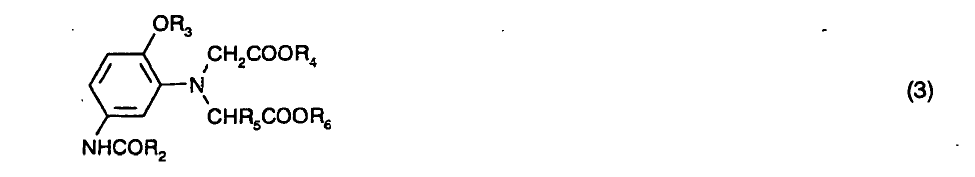 kuppelt, wobei R, R₁, R₂, R₃, R₄, R₅ und R₆ die unter der Formel (1) angegebenen Bedeutungen aufweisen.

Die Diazotierung der Verbindungen der Formel (2) erfolgt in an sich bekannter Weise, z.B. mit Natriumnitrit in saurem, z.B. salzsaurem oder schwefelsaurem, wässrigem Medium. Die Diazotierung kann aber auch mit anderen Diazotierungsmitteln, z.B. mit Nitrosylschwefelsäure ausgeführt werden. Bei der Diazotierung kann eine zusätzliche Säure im Reaktionsmedium anwesend sein, z.B. Phosphorsäure, Schwefelsäure, Essigsäure, Propionsäure, Salzsäure oder Mischungen dieser Säuren, z.B. Mischungen aus Phosphorsäure und Essigsäure. Zweckmässig wird die Diazotierung bei Temperaturen von -10 bis 30° C, vorzugsweise von -10° C bis Raumtemperatur, durchgeführt.

Die Kupplung der diazotierten Verbindung der Formel (2) auf die Kupplungskomponente der Formel (3) erfolgt ebenfalls in bekannter Weise, beispielsweise in saurem, wässrigem oder wässrig-organischem Medium, vorteilhaft bei Temperaturen von -10 bis 30°C, insbesondere unter 10° C. Als Säuren verwendet man z.B. Salzsäure, Essigsäure, Schwefelsäure oder Phosphorsäure. Diazotierung und Kupplung können beispielsweise im gleichen Reaktionsmedium durchgeführt werden.

Die Diazokomponenten der Formel (2) und die Kupplungskomponenten der Formel (3) sind z. T. bekannt oder können auf an sich bekannte Art und Weise hergestellt werden.

Die Kupplungskomponente der Formel 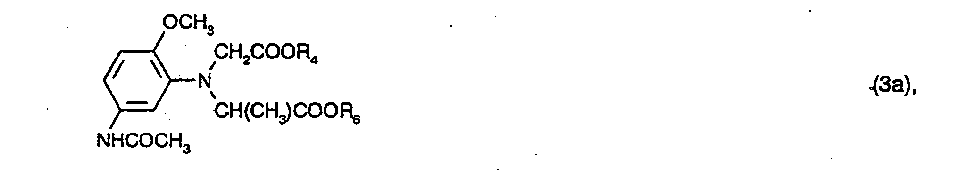 worin R₄ und R₆ die unter der Formel (1) angegebene Bedeutung haben, ist neu und stellt einen weiteren Gegenstand der vorliegenden Erfindung dar.

Die Kupplungskomponente der Formel (3a) wird z.B. so hergestellt, dass man 3-Amino-4-methoxy-acetanilid zuerst mit einer Verbindung der Formel CH₃-CHCl-COOR₆ und anschliessend mit einer Verbindung der Formel CH₂Cl-COOR₄ umsetzt.

Die erfindungsgemässen Farbstoffe der Formel (1) können zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Fasermaterialien enthalten, können ebenfalls mit Hilfe der erfindungsgemässen Farbstoffe gefärbt oder bedruckt werden.

Als halbsynthetische Textilmaterialien kommen vor allem Cellulose-2½-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-cyclohexan; aus Polycarbonaten, z.B. solchen aus α,α-Dimethyl-4,4'-dihydroxy-diphenylmethan und Phosgen, aus Fasern auf Polyvinylchlorid- sowie Polyamid-Basis.

Die Applikation der erfindungsgemässen Farbstoffe auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nichtionischen Dispergiermitteln und gegebenenfalls üblichen Quellmitteln (Carrier) bei Temperaturen zwischen 80 und 140°C. Cellulose-2½-acetat färbt man vorzugsweise zwischen ungefähr 65 bis 85°C und Cellulosetriacetat bei Temperaturen bis zu 115°C.

Die erfindungsgemässen Farbstoffe färben im Färbebad gleichzeitig anwesende Wolle und Baumwolle nicht oder nur wenig an (sehr gute Reserve), so dass sie auch gut zum Färben von Polyester/Wolle- und Polyester/Cellulosefaser-Mischgeweben verwendet werden können.

Die erfindungsgemässen Farbstoffe eignen sich zum Färben nach dem Thermosolverfahren, im Auszieh- und Continueverfahren und für Druckverfahren. Das Ausziehverfahren ist bevorzugt. Das Flottenverhältnis ist von den apparativen Gegebenheiten, vom Substrat und der Aufmachungsform abhängig. Es kann jedoch innerhalb eines weiten Bereiches gewählt werden, z.B. 1:4 bis 1:100, liegt aber vorzugsweise zwischen 1:6 bis 1:25.

Das genannte Textilmaterial kann dabei in den verschiedenen Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat zu überführen. Hierzu wird der Farbstoff vermahlen, so dass seine Teilchengrösse im Mittel zwischen 0,1 und 10 Mikron beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser Druckpasten und Färbebäder herstellen.

Gegenstand der vorliegenden Erfindung sind weiterhin Farbstoffzusammensetzungen, dadurch gekennzeichnet, dass sie
a) als Farbstoffkomponente 30 bis 50 Gew.-%, bezogen auf das Gesamtgewicht der Farbstoffzusammensetzung, eines Farbstoffs der Formel 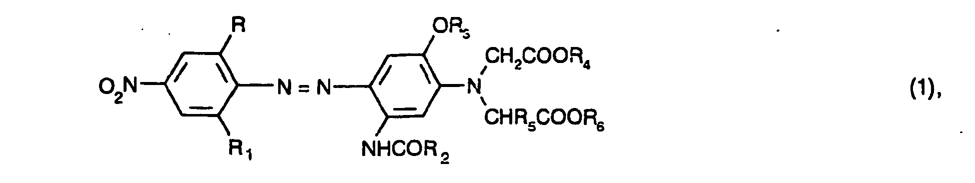 worin R Nitro oder Cyano, R₁ Halogen, R₂ unsubstituiertes, oder durch C₁-C₃-Alkoxy, Halogen, Cyano oder Phenyl substituiertes C₁-C₄-Alkyl, R₃ C₁-C₄-Alkyl, R₄ Methyl oder Ethyl, R₅ Methyl und R₆ Methyl oder Ethyl sind, und
b) 50 bis 70 Gew.-%, bezogen auf das Gesamtgewicht der Farbstoffzusammensetzung, eines Dispergiermittels enthalten.

Als Dispergiermittel kommen beispielsweise anionische Dispergiermittel, wie aromatische Sulfonsäure-Formaldehydkondensate, sulfonierte Creosot Öl-Formaldehyd Kondensate, Ligninsulfonate oder Copolymerisate aus Acrylsäure- und Styrolderivaten, vorzugsweise aromatische Sulfonsäure-Formaldehydkondensate oder Ligninsulfonate, oder nichtionogene Dispergiermittel auf der Basis von Polyalkylenoxiden, die z.B. durch Polyadditionsreaktion aus Ethylenoxid oder Propylenoxid erhältlich sind, in Frage.

Bevorzugt sind die erfindungsgemässen Farbstoffzusammensetzungen fest.

Die erfindungsgemässen Farbstoffzusammensetzungen zeichnen sich dadurch aus, dass sie sich leicht in die applikationsfähige Form, wie beispielsweise in die fertige Druckpaste oder die fertigen Färbebader, überführen lassen.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere, oder Polyvinylalkohole.

Die erfindungsgemässen Farbstoffe verleihen den genannten Materialien, vor allem dem Polyestermaterial, egale blaue bis marineblaue Farbtöne von sehr guten Gebrauchsechtheiten, wie guter Lichtechtheit und guter Sublimierechtheit. Hervorzuheben ist die sehr gute Wasch- und Schweissechtheit und vor allem Thermomigrierechtheit. Die erfindungsgemässen Farbstoffe zeichnen sich ferner durch einen guten Auszug und Aufbau aus.

Die erfindungsgemässen Farbstoffe können auch gut verwendet werden zur Herstellung von Mischnuancen sowohl untereinander als auch zusammen mit anderen Farbstoffen.

Die vorstehend genannten Verwendungen der erfindungsgemässen Farbstoffe stellt ebenso einen Gegenstand der vorliegenden Erfindung dar, wie ein Verfahren zum Färben oder Bedrucken von halbsynthetischem oder synthetischem hydrophobem Fasermaterial, insbesondere Textilmaterial, das darin besteht einen oder mehrere erfindungsgemässen Farbstoffe auf das genannte Material aufzubringen oder es in dieses einzuarbeiten. Das genannte hydrophobe Fasermaterial ist vorzugsweise textiles Polyestermaterial. Weitere Substrate, die durch das erfindungsgemässe Verfahren behandelt werden können sowie bevorzugte Verfahrensbedingungen sind vorstehend bei der näheren Erläuterung der Verwendung der erfindungsgemässen Farbstoffe zu finden.

Ein weiterer Gegenstand der Erfindung ist das durch das genannte Verfahren gefärbte bzw. bedruckte hydrophobe Fasermaterial, vorzugsweise Polyester-Textilmaterial.

Die erfindungsgemässen Farbstoffe der Formel (1) eignen sich ausserdem für moderne Aufzeichnungsverfahren, wie z.B. Thermotransfer-Printing.

Die nachfolgenden Beispiele dienen der Veranschaulichung der Erfindung. Darin sind, sofern nicht anders angegeben, die Teile Gewichtsteile und die Prozente Gewichtsprozente. Die Temperaturen sind in Celsiusgraden angegeben. Die Beziehung zwischen Gewichtsteilen und Volumenteilen ist dieselbe wie zwischen Gramm und Kubikzentimeter.

### Beispiel 1:

60,0 Gewichtsteile 3-Amino-4-methoxy-acetanilid werden bei einer Temperatur von 20 bis 30°C in 200 Gewichtsteile Chloressigsäure-methylester in einem Reaktionskolben eingetragen. Danach werden 60 Gewichtsteile Natriumcarbonat zugegeben. Die entstandene

Suspension wird gleichmässig unter ständigem Rühren auf 115° C aufgeheizt und 6 Stunden bei dieser Temperatur gehalten. Danach wird die ausreagierte Mischung auf Raumtemperatur abgekühlt, mit 330 Gewichtsteilen Wasser versetzt und 30 Minuten verrührt, bis die Salze vollständig gelöst sind. Nach anschliessendem kurzen Stehenlassen bilden sich in dem Reaktionskolben 2 Phasen. Die untere, organische Phase wird abgetrennt. Am Rotationsverdampfer wird aus der organischen Phase das überschüssige Chloressigsäure-methylester abdestilliert. Die erhaltenen 105 Gewichtsteile der Verbindung der Formel 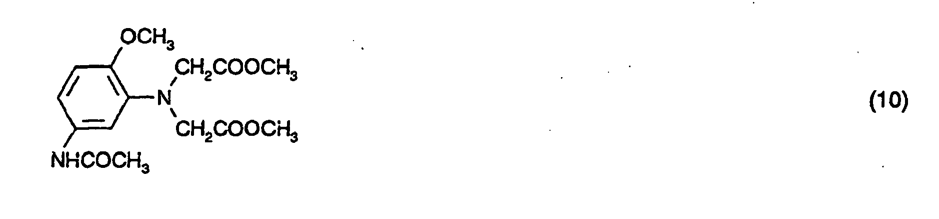 in Form eines harzigen Rückstandes werden in 195 Gewichtsteilen Essigsäure gelöst.

### Beispiel 2: (nicht erfindungsgemäß)

In einem Reaktionskolben werden 72,6 Gewichtsteile 2-Amino-3-brom-5-nitrobenzonitril in 107 Gewichtsteilen 98%-iger Schwefelsäure bei einer maximalen Temperatur von 35°C gelöst. Danach werden bei einer Temperatur zwischen 25 und 28° C 104 Gewichtsteile 40%-iger Nitrosylschwefelsäure innerhalb 40 Minuten zum Reaktionsgemisch zugetropft und 120 Minuten bei 25°C nachgerührt. Die entstandene Diazolösung wird anschliessend innerhalb 60 Minuten bei 0 bis 5° C zu 300 Gewichtsteilen der 35%-iger Lösung der Kupplungskomponente aus Beispiel 1, und 200 Gewichtsteilen Eis zugetropft, wobei die Reaktionstemperatur durch Zugabe von Eis bei maximal 5°C gehalten wird. Nach der beendeten Zugabe der Diazolösung wird das Gemisch 2 Stunden nachgerührt, wobei die Temperatur auf 20°C ansteigt.

Der entstandene Niederschlag wird abfiltriert und mit Wasser gewaschen. Nach dem Trocknen erhält man 153 Gewichtsteile eines Farbstoffes der Formel 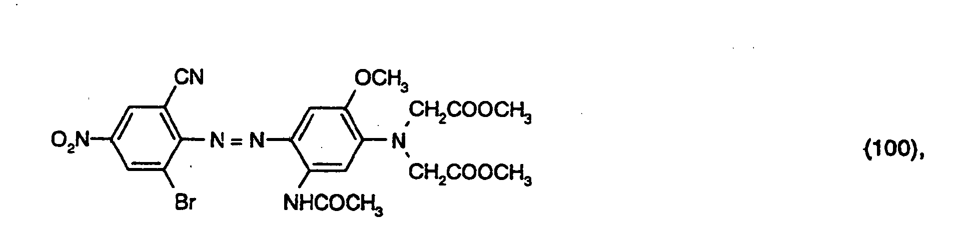 der Textilmaterial aus Polyester in einem blauen Farbton mit guten Echtheiten, insbesondere Thermomigrierechtheit und Waschechtheit, färbt.

### Beispiele 3 - 22:

Analog der im Beispiel 2 angegebenen Vorschrift lassen sich die in der Tabelle 1 aufgeführten Farbstoffe der Formeln (101) bis (120) herstellen. Sie färben ebenfalls das Textilmaterial aus Polyester in marineblauen und blauen Farbtönen mit guten Echtheiten, insbesondere Thermomigrierechtheit und Waschechtheit.

**Tabelle 1**

| | | | | | | |
|---|---|---|---|---|---|---|
| 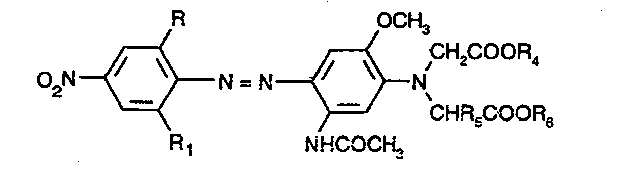 | | | | | | |

| Bsp. Nr./ Formel | R | R₁ | R₄ Nr. | R₅ | R₆ | Nuance |
|---|---|---|---|---|---|---|
| 3 / (101) | NO₂ | Br | CH₃ | CH₃ | CH₃ | marineblau |
| 4 / (102) | NO₂ | Br | CH₂CH₃ | CH₃ | CH₃ | marineblau |
| 5 / (103) | NO₂ | Br | CH₃ | CH₃ | CH₂CH₃ | marineblau |
| 6 / (104) | NO₂ | Br | CH₂CH₃ | CH₃ | CH₂CH₃ | marineblau |
| 7 / (105) | NO₂ | Cl | CH₃ | CH₃ | CH₃ | marineblau |
| 8 / (106) | NO₂ | Cl | CH₃ | CH₃ | CH₂CH₃ | marineblau |
| 9 / (107) | NO₂ | Cl | CH₂CH₃ | CH₃ | CH₃ | marineblau |
| 10 / (108) | NO₂ | Cl | CH₂CH₃ | CH₃ | CH₂CH₃ | marineblau |
| 11 / (109) | CN | Br | CH₂CH₃ | H | CH₂CH₃ | blau |
| 12 / (110) | CN | Br | CH₃ | CH₃ | CH₃ | blau |
| 13 / (111) | CN | Br | CH₃ | CH₃ | CH₂CH₃ | blau |
| 14 / (112) | CN | Br | CH₂CH₃ | CH₃ | CH₃ | blau |
| 15 / (113) | CN | Br | CH₂CH₃ | CH₃ | CH₂CH₃ | blau |
| 16 / (114) | CN | Cl | CH₃ | H | CH₃ | blau |
| 17 /(115) * | CN | Cl | CH₂CH₃ | H | CH₃ | blau |
| 18 / (116) * | CN | Cl | CH₂CH₃ | H | CH₂CH₃ | blau |
| 19 / (117) | CN | Cl | CH₃ | CH₃ | CH₃ | blau |
| 20 / (118) | CN | Cl | CH₂CH₃ | CH₃ | CH₃ | blau |
| 21 / (119) | CN | Cl | CH₃ | CH₃ | CH₂CH₃ | blau |
| 22 / (120) | CN | Cl | CH₂CH₃ | CH₃ | CH₂CH₃ | blau |

| | | | | | | |
|---|---|---|---|---|---|---|
| * nicht erfindungsgemäß | | | | | | |

Bevorzugt sind Farbstoffe der Formeln (101) bis (108), (110) bis (113) und (117) bis (120). Besonders bevorzugt sind Farbstoffe der Formeln (106), (108), (110), (117) und (119).

## Patentansprüche

1. Farbstoffe der Formel 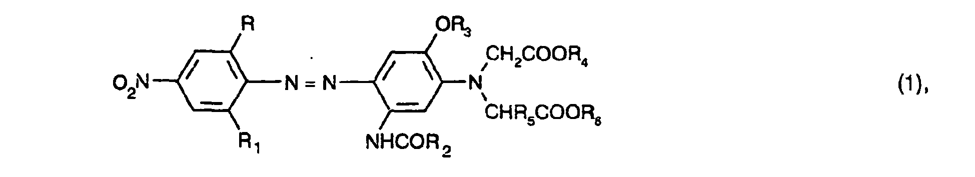 worin R Nitro oder Cyano, R₁ Halogen, R₂ unsubstituiertes, oder durch C₁-C₃-Alkoxy, Halogen, Cyano oder Phenyl substituiertes C₁-C₄-Alkyl, R₃ C₁-C₄-Alkyl, R₄ Methyl oder Ethyl, R₅ Methyl und R₆ Methyl oder Ethyl sind.

2. Farbstoffe gemäss Anspruch 1, worin R₁ Chlor oder Brom ist.

3. Farbstoffe gemäss Anspruch 1 oder 2, worin R₂ Ethyl oder Methyl ist.

4. Farbstoffe gemäss einem der Ansprüche 1 bis 3, worin R₃ Ethyl oder Methyl ist.

5. Farbstoffe gemäss Anspruch 1, worin
R Cyano, R₁ Chlor oder Brom, R₂ und R₃ unabhängig voneinander Methyl oder Ethyl, R₄ Methyl oder Ethyl, R₅ Methyl und R₆ Methyl oder Ethyl sind.

6. Verfahren zur Herstellung von Farbstoffen der Formel (1) gemäss Anspruch 1, **dadurch gekennzeichnet, dass** man eine Verbindung der Formel 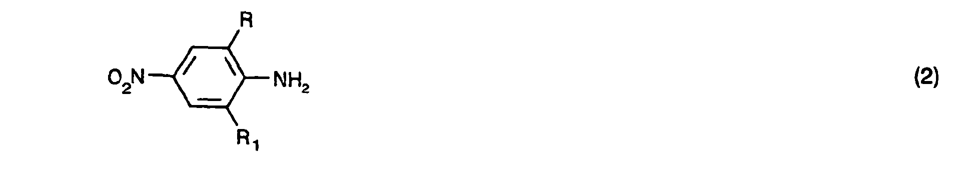 diazotiert und auf eine Kupplungskomponente der Formel 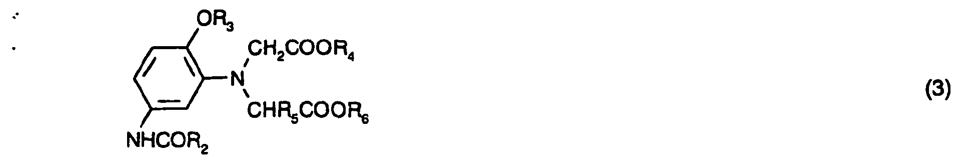 kuppelt, wobei R, R₁, R₂, R_{3,} R₄, R₅ und R₆ die unter der Formel (1) angegebenen Bedeutungen aufweisen.

7. Verfahren zum Färben oder Bedrucken von halbsynthetischem oder synthetischem hydrophobem Fasermaterial, insbesondere Textilmaterial, **dadurch gekennzeichnet, dass** man einen oder mehrere Farbstoffe gemäss Anspruch 1 auf das genannte Material aufbringt oder diesem einverleibt.

8. Verfahren nach Anspruch 7, worin das hydrophobe Material, vorzugsweise Textilmaterial, aus Polyesterfasern besteht.

9. Das gemäss Anspruch 7 oder 8 gefärbte oder bedruckte Material.

10. Kupplungskomponente der Formel 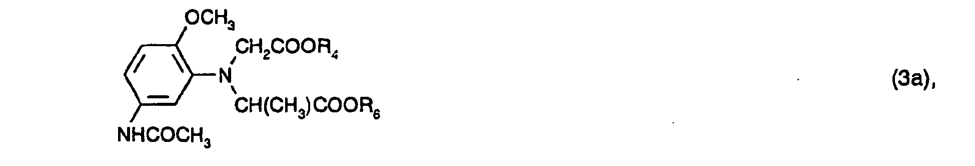 worin R₄ Methyl oder Ethyl und R₆ Methyl oder Ethyl sind.

11. Verfahren zur Herstellung der Kupplungskomponente der Formel (3a) gemäss An-spruch 10, **dadurch gekennzeichnet, dass** man 3-Amino-4-methoxy-acetanilid zuerst mit einer Verbindung der Formel CH₃-CHCl-COOR₆ und anschliessend mit einer Verbindung der Formel CH₂Cl-COOR₄ umsetzt, wobei R₄ und R₆ die unter der Formel (3a) angegebene Bedeutung haben.

12. Farbstoffzusammensetzungen, **dadurch gekennzeichnet, dass** sie
a) als Farbstoffkomponente 30 bis 50 Gew.-%, bezogen auf das Gesamtgewicht der Farbstoffzusammensetzung, eines Farbstoffs der Formel 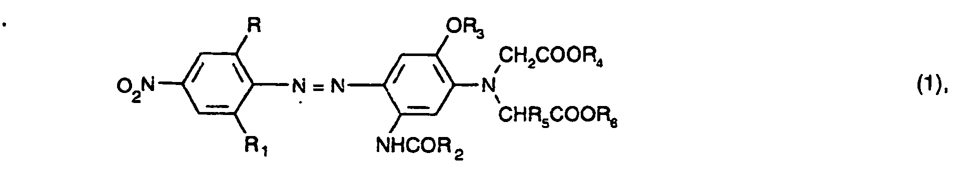 worin R Nitro oder Cyano, R₁ Halogen, R₂ unsubstituiertes, oder durch C₁-C₃-Alkoxy, Halogen, Cyano oder Phenyl substituiertes C₁-C₄-Alkyl, R₃ C₁-C₄-Alkyl, R₄ Methyl oder Ethyl, R₅ Methyl und R₆ Methyl oder Ethyl sind, und
b) 50 bis 70 Gew.-%, bezogen auf das Gesamtgewicht der Farbstoffzusammensetzung, eines Dispergiermittels enthalten.

## Claims

1. Dyes of formula 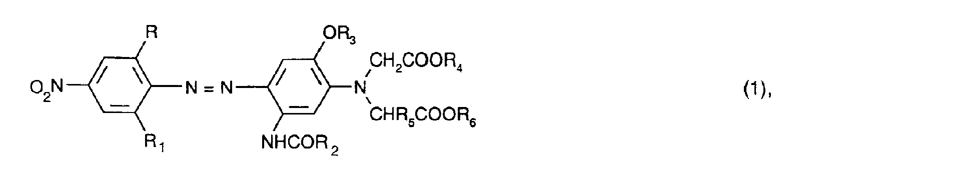 wherein R is nitro or cyano, R₁ is halogen, R₂ is C₁-C₄alkyl unsubstituted or substituted by C₁-C₃alkoxy, halogen, cyano or by phenyl, R₃ is C₁-C₄alkyl, R₄ is methyl or ethyl, R₅ is methyl and R₆ is methyl or ethyl.

2. Dyes according to claim 1, wherein R₁ is chlorine or bromine.

3. Dyes according to claim 1 or 2, wherein R₂ is ethyl or methyl.

4. Dyes according to any one of claims 1 to 3, wherein R₃ is ethyl or methyl.

5. Dyes according to claim 1, wherein
R is cyano, R₁ is chlorine or bromine, R₂ and R₃ are each independently of the other methyl or ethyl, R₄ is methyl or ethyl, R₅ is methyl and R₆ is methyl or ethyl.

6. Process for the preparation of dyes of formula (1) according to claim 1, **characterised in that** a compound of formula 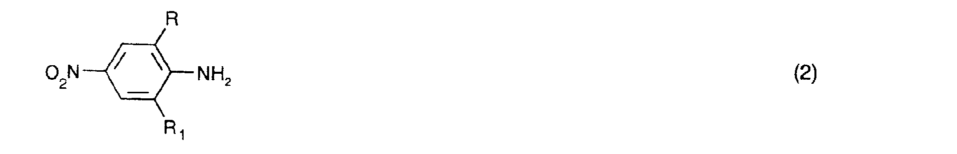 is diazotised and coupled to a coupling component of formula 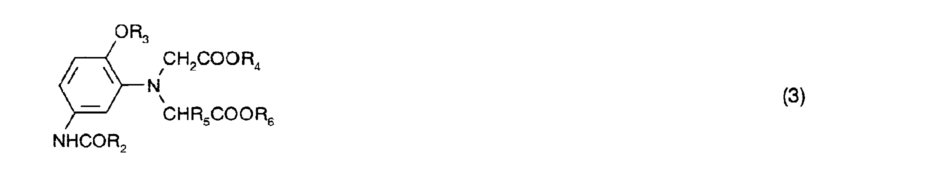 wherein R, R₁, R₂, R₃, R₄, R₅ and R₆ are as defined for formula (1).

7. Method of dyeing or printing semi-synthetic or synthetic hydrophobic fibre material, especially textile material, **characterised in that** one or more dyes according to claim 1 are applied to the said material or incorporated therein.

8. Method according to claim 7, wherein the hydrophobic material, especially textile material, consists of polyester fibres.

9. The material dyed or printed according to claim 7 or 8.

10. Coupling component of formula 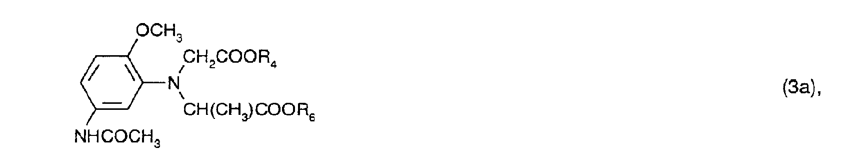 wherein R₄ is methyl or ethyl and R₆ is methyl or ethyl.

11. Process for the preparation of the coupling component of formula (3a) according to claim 10, **characterised in that** 3-amino-4-methoxyacetanilide is reacted first with a compound of formula CH₃-CHCl-COOR₆ and then with a compound of formula CH₂Cl-COOR₄, wherein R₄ and R₆ are as defined for formula (3a).

12. Dye compositions, **characterised in that** they comprise
a) as dye component from 30 to 50 % by weight, based on the total weight of the dye composition, of a dye of formula 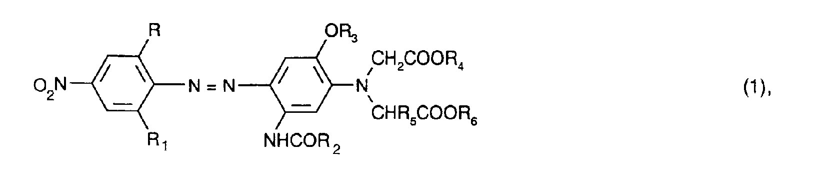 wherein R is nitro or cyano, R₁ is halogen, R₂ is C₁-C₄alkyl unsubstituted or substituted by C₁-C₃alkoxy, halogen, cyano or by phenyl, R₃ is C₁-C₄alkyl, R₄ is methyl or ethyl, R₅ is methyl and R₆ is methyl or ethyl, and
b) from 50 to 70 % by weight, based on the total weight of the dye composition, of a dispersant.

## Revendications

1. Colorants de formule : 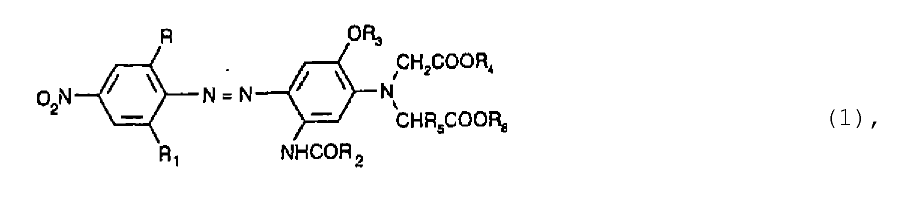 dans laquelle R représente nitro ou cyano, R₁ représente halogène, R₂ représente alkyle en C₁-C₄ non substitué ou substitué par alcoxy en C₁-C₃, halogène, cyano ou phényle, R₃ représente alkyle en C₁-C₄, R₄ représente méthyle ou éthyle, R₅ représente méthyle et R₆ représente méthyle ou éthyle.

2. Colorants selon la revendication 1, dans lesquels R₁ représente chlore ou brome.

3. Colorants selon l'une des revendications 1 ou 2, dans lesquels R₂ représente éthyle ou méthyle.

4. Colorants selon l'un des revendications 1 à 3, dans lesquels R₃ représente éthyle ou méthyle.

5. Colorants selon la revendication 1, dans lesquels R représente cyano, R₁ représente chlore ou brome, R₂ et R₃ indépendamment l'un de l'autre représentent méthyle ou éthyle, R₄ représente méthyle ou éthyle, R₅ représente méthyle et R₆ représente méthyle ou éthyle.

6. Procédé de fabrication de colorants de la formule (1) selon la revendication 1, **caractérisé par le fait que** l'on conduit une diazotation d'un composé de la formule : 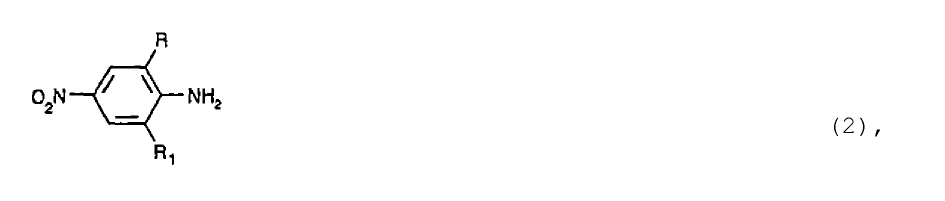 et que l'on conduit un couplage sur un composant de couplage de la formule : 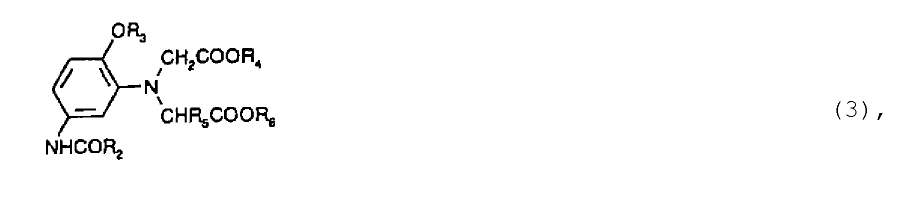 où R, R₁, R₂, R₃, R₄, R₅ et R₆ présentent les significations indiquées sous la formule (1).

7. Procédé de teinture ou d'impression de matière fibreuse hydrophobe semi-synthétique ou synthétique, en particulier de matière textile, **caractérisé par le fait que** l'on applique ou que l'on fait absorber un ou plusieurs colorants selon la revendication 1 sur la matière précitée ou dans celle-ci.

8. Procédé selon la revendication 7, dans lequel la matière hydrophobe, de préférence une matière textile, se compose de fibres de polyester.

9. Matière teinte ou imprimée selon la revendication 7 ou 8.

10. Composant de couplage de la formule : 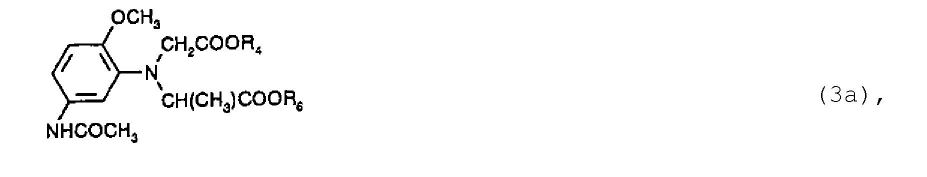 dans laquelle R₄ représente méthyle ou éthyle et R₆ représente méthyle ou éthyle

11. Procédé de fabrication du composant de couplage de la formule (3a) selon la revendication 10, **caractérisé par le fait que** l'on fait réagir du 3-amino-4-méthoxy-acétanilide tout d'abord avec un composé de la formule CH₃-CHCl-COOR₆ puis avec un composé de la formule CH₂Cl-COOR₄, R₄ et R₆ ayant la signification indiquée sous la formule (3a).

12. Compositions de colorant **caractérisées par le fait qu'**elles contiennent :
a) comme composant colorant, 30 à 50 % en poids, par rapport au poids total de la composition de colorant d'un colorant de la formule : 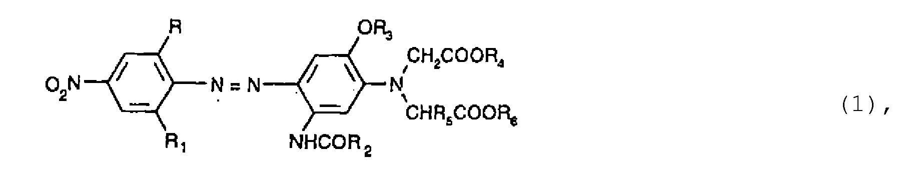 dans laquelle R représente nitro ou cyano, R₁ représente halogène, R₂ représente alkyle en C₁-C₄ non substitué ou substitué par alcoxy en C₁-C₃, halogène, cyano ou phényle, R₃ représente alkyle en C₁-C₄, R₄ représente méthyle ou éthyle, R₅ représente méthyle et R₆ représente méthyle ou éthyle ; et
b) 50 à 70 % en poids, par rapport au poids total de la composition de colorant, d'un agent de dispersion.
